# EUROPEAN PATENT SPECIFICATION

(11) **EP 2 633 873 B1**
(45) Date of publication and mention of the grant of the patent: **11.05.2016**
(21) Application number: 11836516.2
(22) Date of filing: 02.06.2011
(51) Int. Cl.: A61M 5/315, A61M 37/00, A61M 5/145

(54) **DRUG INJECTING APPARATUS FOR USE IN SKIN SURGERY**
MEDIKAMENTENEINSPRITZVORRICHTUNG ZUR VERWENDUNG IN DER HAUTCHIRURGIE
APPAREIL D'INJECTION DE MÉDICAMENT UTILISABLE EN CHIRURGIE DE LA PEAU

(30) Priority: 28.10.2010 KR 20100106297
(43) Date of publication of application: 04.09.2013
(73) Proprietor: Panace Co., Ltd., Seongnam-si, Gyeonggi-do 462-807 (KR)
(72) Inventor: BANG, Si-Yeol, Yongin-si Gyeonggi-do 448-803 (KR)
(74) Representative: Manitz, Finsterwald & Partner GbR
(86) International application number: PCT/KR2011/004028
(87) International publication number: WO 2012/057424

(56) References cited:
- EP-A1- 2 345 441
- WO-A1-2007/012915
- WO-A1-2008/153985
- WO-A1-2009/125879
- GB-A- 2 109 242
- KR-B1- 100 589 077
- KR-B1- 100 745 554
- KR-B1- 100 814 493
- US-A- 4 544 369
- US-A1- 2010 063 447

## Description

### [Technical Field]

The present invention relates to a drug injecting apparatus for use in skin surgery, and more particularly, to a drug injecting apparatus for use in skin surgery according to the preamble of claim 1. Such an apparatus is disclosed in US 4,544,369 A e.g.

Similar apparatus are known from references WO 2009/125 879 A1, WO 2008/153 985 A1, US 2010/063 447 A1, WO 2007/012 915 A1 or GB2 109 242 A.

### [Background Art]

Multi-hole therapy is a therapy for making a plurality of fine holes into the skin and injecting drug into a human body through the holes, and is applicable to various fields, for instance, treatment of various skin troubles, such as wrinkle, freckles, blemishes, stretch marks, pimples, acnes, pigmentation, and so on, hair loss treatment, obesity treatment, etc.

Here, when drug is used for the multi-hole therapy, drug of an accurate amount must be generally injected into the skin several times little by little so as to promote a clear-cut and uniform effect all over the skin. However, because an aesthetician must inject drug depending on muscular power of his or her arm and fingers and his or her feeling that drug is injected into the skin, it is difficult to inject drug of the accurate amount into the skin, and sometimes there is a problem that the expensive drug is wasted due to injection or leakage of a drug overdose.

### [Disclosure]

### [Technical Problem]

Accordingly, the present invention has been made in an effort to solve the above-mentioned problems occurring in the prior arts, and it is an object of the present invention to provide a drug injecting apparatus for use in skin surgery, which can inject drug of an adequate amount into the skin to an accurate depth without abuse and waste of drug.

### [Technical Solution]

To achieve the above objects, the present invention provides a drug injecting apparatus for use in skin surgery including the features of claim 1.

### [Advantageous Effects]

As described above, the drug injecting apparatus for use in skin surgery has following advantages as follows.

First, because the drug injecting apparatus injects drug of an accurate amount into the skin according to the structure that includes the transfer guide assembly for allowing a reciprocating motion of the cylinder of the syringe and the injection assembly for moving the rod of the cylinder, which injects drug, to a proper distance while moving to the needle by the controlling part, the drug injecting apparatus can prevent waste of drug.

That is, when the end portion of the needle gets in contact with the skin and is inserted into the skin, the controlling part senses a movement position of the transfer guide assembly so as to operate or stops the injection assembly, and hence, the present invention can inject drug of the accurate amount into the skin.

Furthermore, according to the preferred embodiment of the present invention, the drug injecting apparatus can accurately regulate the depth of the needle inserted into the skin because the transfer guide assembly adjusts a reciprocation distance of the cylinder.

### [Description of Drawings]

FIG. 1 is a side conceptual view showing the entire structure of a drug injecting apparatus for use in skin surgery according to a first preferred embodiment of the present invention.
FIG. 2 is a sectional conceptual view showing the entire structure of the drug injecting apparatus for use in skin surgery according to the first preferred embodiment of the present invention.
FIG. 3 is a bottom conceptual view showing a structure of a transfer guide assembly viewed at the "A" viewpoint of FIG. 1.
FIG. 4 is a view showing a transfer guide assembly, which is an essential part of a drug injecting apparatus for use in skin surgery according to a second preferred embodiment of the present invention.
FIG. 5 is a view showing a structure of a transfer fragment of a motion transducer of an injection assembly, which is an essential part of a drug injecting apparatus for use in skin surgery according to a second preferred embodiment of the present invention.

### [Best Mode]

A drug injecting apparatus for use in skin surgery according to the present invention for accurately adjusting a depth of a needle inserted into the skin so as to prevent waste of drug includes: a syringe for injecting drug into the skin through a needle disposed at an end portion thereof; a transfer guide assembly joined with a cylinder of the syringe in which drug is contained, the transfer guide assembly allowing the cylinder to carry out a reciprocating motion within a limited range according to a direction that drug is injected; an injection assembly mounted at one side of the transfer guide assembly for moving a rode of the cylinder toward the needle in interlock with the transfer guide assembly while rotating by a driving force; and a controlling part mounted at one side of the transfer guide assembly and the injection assembly, the controlling part serving to operate the injection assembly when the transfer guide assembly moves to one end of the limited range in a state where an end portion of the needle gets in contact with the skin and to stop the operation of the injection assembly when the end portion of the needle is separated from the skin and the transfer guide assembly moves to the other end of the limited range, wherein the injection assembly transfers the rod toward the needle as long as the cylinder retracts toward the injection assembly.

### [Mode for Invention]

Reference will be now made in detail to the preferred embodiment of the present invention with reference to the attached drawings.

The preferred embodiments of the present invention will be described in detail with reference to the accompanying drawings so that those skilled in the art can easily implement the technical idea of the present invention, but it does not mean that the preferred embodiments do not limit the technical idea and scope of the present invention.

Moreover, it will be understood that sizes or shapes of components illustrated in the drawings may be illustrated exaggeratedly for clarity and for convenience sake in description, and it will be further understood that terms specially defined in consideration of configurations and actions of the present invention may be changed according to users' intentions or according to custom and the definition of the terms and should be interpreted in the context of the contents throughout the specification of the present invention.

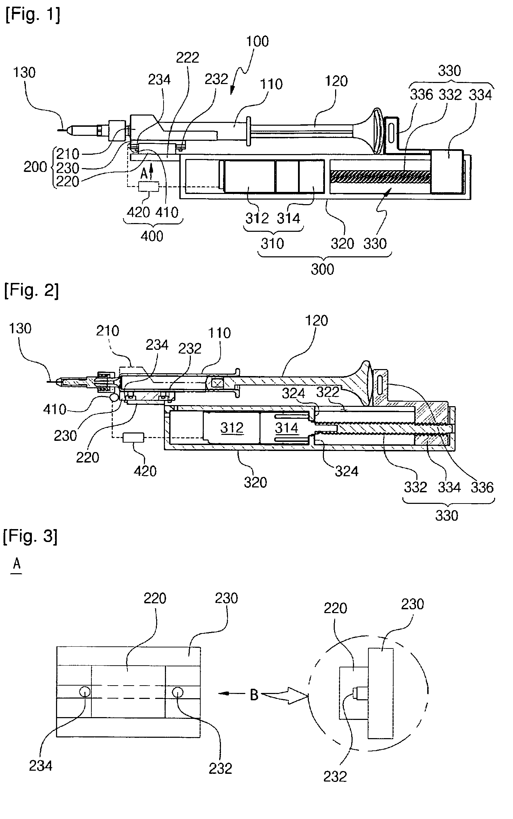

FIG. 1 is a side conceptual view showing the entire structure of a drug injecting apparatus for use in skin surgery according to a first preferred embodiment of the present invention.

As shown in the drawing, the drug injecting apparatus for use in skin surgery according to the present invention includes: a syringe 100; a transfer guide assembly 200 joined to guide a reciprocating motion of the syringe 100; an injection assembly 300 for injecting drug of an accurate amount into the skin; and a controlling part 400 for controlling operation and stop of the injection assembly 300.

First, the syringe 100 is to inject drug into the skin while a rod 120 moves toward a needle 130 through the needle 130 mounted at an end portion of a cylinder 110 in which drug is contained.

The transfer guide assembly 200 is joined with the cylinder 110 of the syringe 100 in which drug is contained and allows the reciprocating motion of the cylinder 110 along a direction that drug is injected within a limited range.

Moreover, the injection assembly 300 is mounted at one side of the transfer guide assembly 200 and is rotated by a driving force in interlock with the transfer guide assembly 200 so as to move the rod 120 of the cylinder 110 toward the needle 130.

Here, the controlling part 400 is mounted at one side of the transfer guide assembly 200 and the injection assembly 300 in order to inject drug of an adequate amount into the skin through the needle 130 while operating the injection assembly 300 when an end portion of the needle 130 is inserted into the skin in contact with the skin and the transfer guide assembly 200 moves to an one side end portion within the limited range but stopping the injection assembly 300 when the end portion of the needle 130 is separated from the skin and the transfer guide assembly 200 moves to the other side end portion within the limited range.

That is, the injection assembly 300 moves the rod 120 toward the needle 130 as long as the cylinder 110 is retracted toward the injection assembly 300, so as to inject drug of an accurate amount into the skin.

The present invention is applicable by the above preferred embodiment, and essential parts of the present invention will be described in more detail.

First, the transfer guide assembly 200, as described above, is joined with the syringe 100 to allow the reciprocating motion of the cylinder 110, in more detail, that the cylinder 110 retracts as long as a predetermined distance in a state where the needle 130 gets in contact with the skin (however, it may be seen that the needle 130 is not retracted because the rod 120 moves forward by the injection assembly 300 in fact). As shown in FIGS. 2 and 3, the transfer guide assembly 200 includes a bracket 210, a support block 220, and a guide rail 230.

For your reference, in FIG. 3, for ease of understanding drawings, the bracket 210 is not illustrated.

The bracket 210 is a member for fixing the cylinder 110, the support block 220 is mounted on a prop fragment 222 mounted at a front end portion of the injection assembly 300, and the guide rail 230 is joined to the bottom of the bracket 210 and coupled with the support block 220 so as to reciprocate the bracket 210.

Here, detents 232 and 234 are disposed at front and rear end portions of the guide rail 230.

The detents 232 and 234 are to limit a reciprocation distance of the bracket 210 according to the movement of the guide rail 230 joined with the support block 220. Of course, the support block 220 and the guide rail 230 may adopt something like an LM guide to accurately regulate a fine movement distance.

Accordingly, a distance (d) between the support block 220, which gets in contact with the detent 232 of one side, and the detent 234 of the other side is equal with the depth that the needle 130 is inserted into the skin.

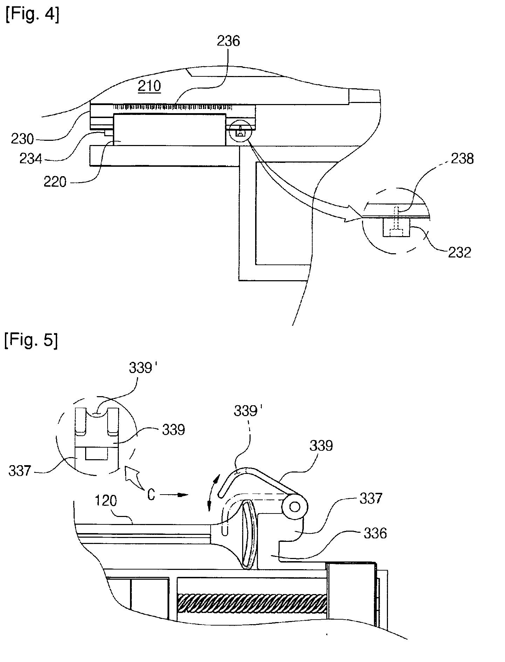

Furthermore, as shown in FIG. 4, the transfer guide assembly 200 has a scale 236 disposed at an edge of the guide rail 230 so as to regulate an accurate movement distance, namely, the depth that the needle 130 is inserted into the skin.

Additionally, the guide rail 230 may further include an adjustable screw 238 for fixing the detent 232 which is reciprocally mounted on the guide rail 220, out of the detents 232 and 234 disposed at the front and rear end portions.

Accordingly, the user can regulate the depth of drug injected into the skin according to kinds of various therapies.

In the meantime, as described above, the injection assembly 300 can make the rod 120 continuously move toward the needle 130 or stop the movement of the rod 120 by the controlling part 400. As shown in FIG. 2, when a driving force transferring member 310 transfers the driving force and is accommodated in a case 320, the rod 120 can continuously move toward the needle 130 or stop the movement by a motion transducer 330.

The driving force transferring member 310 is operated or stopped by the controlling part 400, which will be described later, transfers the driving force to the rod 120, and includes a driving motor 312 and a decelerator 314.

The case 320 has a space for accommodating the driving force transferring member 310, and the transfer guide assembly 200, in detail, the guide rail 220, is mounted at a front end portion of the case 320. The case 320 includes: a movement slit 322 perforated in an upper face along a longitudinal direction of the case 320 in such a manner that the motion transducer 330 moves in the movement slit 322; and a support fragment 324 integrally built in the case 320 for fixing the driving force transferring member 310.

The motion transducer 330 is connected with the driving force transferring member 310, namely, the decelerator 314, and the rod 120, converts a rotary motion into a reciprocating motion in interlock with the driving force transferring member 310 according to the longitudinal direction of the case 320 and moves the rod 120 toward the needle 130, and includes a transfer bar 332, a movement fragment 334, and a transfer fragment 336.

Here, the driving motor 312 of the driving force transferring member 310 is embedded in the case 320 and rotates forward and backward, and the decelerator 314 is mounted between the driving motor 312 and the motion transducer 330 and reduces rpm of the driving motor 312 at a proper speed to gradually move the rod 120 toward the needle 130.

In this instance, the transfer bar 332 of the motion transducer 330 has an end portion joined with an end portion of the driving force transferring member 310 so as to rotate forward and backward, the other end portion supported to the case 320, and a spirally threaded outer circumferential surface so as to provide a movement area of the movement fragment 334, which will be described later.

The movement fragment 334 is screw-coupled with the transfer bar 332 so as to reciprocate along the longitudinal direction of the transfer bar 332, and provides the movement area of the movement fragment 334, which will be described later.

The transfer fragment 336 is joined to the movement fragment 334 and exposed to the upper portion of the case 320 so as to abut on the rod 120. The transfer fragment 336 serves to move the rod 120 toward the needle 130 while moving along the movement slit 322 of the case 320.

Moreover, as shown in FIG. 5, the transfer fragment 336 may include a fixing fragment 339, which extends from the edge of the transfer fragment 336, is rotatably joined with an end portion of a body 337 having the side on which the end portion of the rod 120 abuts, and has a fixing slit 339' disposed at an end portion thereof and having a width corresponding with the outer circumference of the rod 120.

Accordingly, the transfer fragment 336 can move the rod 120 toward the needle 130 without any vibration while keeping the state where the fixing fragment 339 is firmly joined with the rod 120.

In the meantime, as described above, the controlling part 400 is to control the operation of the injection assembly 300. As shown in FIGS. 1 and 2, when the end portion of the needle 130 is inserted into the skin in contact with the skin by sensing means 410, according to whether the support block 220 moves to the end portion of one side of the guide rail 230 or moves to the end portion of the other side of the guide rail 230, a controller 420 can control rotation or stop of the injection assembly, namely, the driving motor 312 of the driving force transferring member 310.

In other words, the sensing means 410 is mounted at a front end portion of the transfer guide assembly 200, namely, the guide rail 220, and transfers a sensing signal on that the front end portion (the left side in the drawings) of the guide rail 230 gets in contact with the support block 220.

Here, in the drawings, it is illustrated that the sensing means 410 is mounted on the front end portion of the guide rail 220, but the present invention is not restricted to the above, and the sensing means 410 may be a spring-embedded switch mounted at the rear end portion of the guide rail 220, a limit switch, a position detection sensor, a pressure sensor, or others.

In this instance, the sensing means 410 may be respectively mounted at the front end portion and the rear end portion of the guide rail 220 in order to transfer the signal more accurately.

Therefore, as an example of the sensing means 410, if such a spring-embedded switch is mounted at the rear end portion of the guide rail 220, when the guide rail 220 moves so as to get in contact with the support block 220, the spring-embedded switch senses the contact action, so that the entire circuit is connected or released, and accordingly, a signal transfer system for operating the injection assembly 300 is completed.

Moreover, the controller 420 is mounted on one side of the injection assembly 300, namely, the case 320 in such a way as to be electrically connected with the sensing means 410 and the driving motor 312 of the injection assembly 300, and transfers an electric signal so that the driving motor 312 is operated or stopped according to the position of the guide rail 230 sensed by the sensing means 410.

Therefore, with reference to the mechanism of the controlling part 400, surgical procedures of the drug injecting apparatus for use in skin surgery according to the present invention will be described by two surgical therapy methods in brief.

First, in relation with the skin therapy that drug of a sufficient amount must be injected throughout the skin for a sufficient period of time, for instance, the skin therapy for previously injecting drug in order to perform liposuction of lipectomy of the abdominal region, the thigh, or the buttocks, the surgical procedures will be described.

In this instance, when the end portion of the needle 130 is inserted into the skin in contact with the skin by the user's manipulation, the sensing means 410 senses that the front end portion of the guide rail 230 gets in contact with the support block 220, and then, the controller 420 transfers an operation signal to the driving motor 312 of the injection assembly 300 so that the rod 120 injects drug while moving forward toward the needle 130.

When the needle 130 is in the state where it is inserted into the skin, the injection assembly 300 is continuously operated and injects drug of the cylinder 110 through the needle 130.

After that, when the user judges that drug of an adequate amount has been injected, and separates the needle 130 from the skin, the front end portion of the guide rail 230 is separated from the support block 220, and then, the sensing means 410 senses it and transfers a signal to stop the operation of the driving motor 312 by the controller 420.

Finally, because the guide rail 230 is returned to its original position, the above-mentioned mechanism is repeated till injection of drug of the cylinder 110 is finished.

Second, in relation with the skin therapy that drug of a small amount is rapidly and densely injected into the skin, for instance, into a small area of a subject's face, the surgical procedures will be described.

In this instance, it is supposed the depth that the end portion of the needle 130 is inserted into the skin is 1mm, namely, the distance that the guide rail 230 reciprocates based on the support block 220 is 1mm.

First, when the end portion of the needle 130 is inserted into the skin by the user's manipulation, the sensing means 410 senses that the front end portion of the guide rail 230 gets in contact with the support block 220, and then, the controller 420 transfers an operation signal to the driving motor 312 of the injection assembly 300 so that the rod 120 injects drug while moving forward toward the needle 130.

Here, the operation signal is to move the rod 120 toward the needle 130, for instance, to 0.5mm, and to stop the movement instead of continuously operating the driving motor 312.

In this instance, ①after the user injects drug of a small amount into the subject's skin and separates the needle 130 from the skin, ②when the user inserts the needle 130 into a portion adjacent to the portion where drug is injected, the sensing means 410 transfers the signal to stop the operation of the driving motor 312 through the controller 420 according to the action ①.

Furthermore, the sensing means 410 transfers the signal to operate the driving motor 312 so that the rod 120 moves forward toward the needle 130 to a distance that 1/2 of the entire reciprocation distance, namely, 0.5mm of 1mm, is divided into plural parts within a reset range.

Accordingly, drug contained in the cylinder 110 can be rapidly and densely injected little by little within a small range of the skin while a series of the above procedures are repeated.

As described above, the basic technical idea of the present invention is to provide the drug injecting apparatus for use in skin surgery, which can inject drug of an adequate amount into the skin to an accurate depth without abuse and waste of drug.

As described above, while the present invention has been particularly shown and described with reference to the example embodiments thereof, it will be understood by those of ordinary skill in the art that the above embodiments of the present invention are all exemplified, the drug injecting apparatus for use in skin surgery according to various embodiments may be used for use in general injection therapy, and various changes, modifications and equivalents may be made therein without departing from the scope of the present invention.

Therefore, it would be understood that the technical and protective scope of the present invention shall be defined by the technical idea as defined by the following claims.

### [Industrial Applicability]

As described above, the present invention relates to a drug injecting apparatus for use in skin surgery, which can inject drug of an adequate amount into the skin to an accurate depth without abuse and waste of drug, is a therapy for making a plurality of fine holes into the skin and injecting drug into a human body through the holes, and is applicable to various fields, for instance, treatment of various skin troubles, such as wrinkle, freckles, blemishes, stretch marks, pimples, acnes, pigmentation, and so on, hair loss treatment, obesity treatment, etc.

## Claims

1. A drug injecting apparatus for use in skin surgery comprising:
a syringe (100) for injecting drug into the skin through a needle (130) disposed at an end portion thereof;
a transfer guide assembly (200) joined with a cylinder (110) of the syringe (100) in which drug is contained, the transfer guide assembly (200) allowing the cylinder (110) to carry out a reciprocating motion within a limited range according to a direction that drug is injected;
an injection assembly (300) mounted at one side of the transfer guide assembly (200) for moving a rod (120) of the cylinder (110) toward the needle (130) in interlock with the transfer guide assembly (200) while rotating by a driving force; and
a controlling part (400) mounted at one side of the transfer guide assembly (200) and the injection assembly (300), the controlling part (400) serving to operate the injection assembly (300) when the transfer guide assembly (200) moves to one end of the limited range in a state where an end portion of the needle (130) gets in contact with the skin and to stop the operation of the injection assembly (300) when the end portion of the needle (130) is separated from the skin and the transfer guide assembly (200) moves to the other end of the limited range,
wherein the injection assembly (300) transfers the rod (120) toward the needle (130) as long as the cylinder (110) retracts toward the injection assembly (300),
**characterized in that**
the transfer guide assembly (200) comprises:
a bracket (210) on which the cylinder (110) is fixed;
a support block (220) mounted on a prop segment (222) mounted at a front end portion of the injection assembly (300); and
a guide rail (230) joined to the bottom of the bracket (210) and coupled with the support block (220), the guide rail (230) having detents (232, 234) respectively disposed at front and rear end portions thereof, so as to reciprocate the bracket (210),
wherein a distance between the support block (220), which gets in contact with the detent (232) of one side, and the detent (234) of the other side is equal with the depth that the needle (130) is inserted into the skin.

2. The drug injecting apparatus according to claim 1, wherein the injection assembly (300) comprises:
a driving force transferring member (310) operated or stopped by the controlling part (400) and transferring a driving force to the rod (120);
a case (320) having a space in which the driving force transferring member (310) is embedded and a front end portion to which the transfer guide assembly (200) is mounted; and
a motion transducer (330) connected with the driving force transferring member (310) and the rod (120) for converting a rotary motion into a reciprocating motion according to a longitudinal direction of the case (320) in interlock with the driving force transferring member (310) and for moving the rod (120) toward the needle.

3. The drug injecting apparatus according to claim 1, wherein the controlling part (400) comprises:
sensing means (410) mounted at a front end portion of the transfer guide assembly (200) for moving the transfer guide assembly (200) to one end of the limited range when the needle (130) gets in contact with the skin and is inserted into the skin or for sensing that the needle (130) is separated from the skin; and
a controller (420) mounted at one side of the injection assembly (300) and electrically connected with the sensing means (410) and the injection assembly (300) so as to transfer an electric signal to the injection assembly (300) so that the injection assembly (300) is operated or stopped according to whether or not the sensing means (410) senses the signal.

4. The drug injecting apparatus according to claim 1, wherein the transfer guide assembly (200) comprises:
a scale (236) disposed at an edge of the guide rail (230); and
an adjustable screw (238) screw-coupled with the guide rail (230) for fixing the detent (232, 234), which is reciprocally mounted on the guide rail (230), out of the two detents (232, 234) disposed at the front and rear end portions.

5. The drug injecting apparatus according to claim 2, wherein the driving force transferring member (410) comprises:
a driving motor (312) embedded in the case (320) and rotating forward and backward; and
a decelerator (314) mounted between a driving shaft of the driving motor (312) and the motion transducer (330).

6. The drug injecting apparatus according to claim 2, wherein the case (320) comprises:
a movement slit (322) perforated in an upper face along a longitudinal direction of the case (320) in such a manner that the motion transducer (330) moves in the movement slit (322); and
a support fragment (324) integrally embedded in the case (320) for fixing the driving force transferring member (410).

7. The drug injecting apparatus according to claim 2, wherein the motion transducer (330) comprises:
a transfer bar (332) having an end portion joined with an end portion of the driving force transferring member (410) so as to rotate forward and backward, the other end portion supported to the case (320), and a spirally threaded outer circumferential surface;
a movement fragment (334) screw-coupled with the transfer bar (332) so as to reciprocate along a longitudinal direction of the transfer bar (332); and
a transfer fragment (336) joined to the movement fragment (334) and exposed to the upper portion of the case (320) so as to abut on the rod.

8. The drug injecting apparatus according to claim 7, wherein the transfer fragment (336) comprises:
a body (337) extending from an edge of the movement fragment (334), the end portion of the rod (120) abutting on the side of the body (337); and
a fixing fragment (339) rotatably joined with an end portion of the body (337), the fixing fragment (339) having a fixing slit (339') formed at an end portion thereof in such a way as to have a width corresponding to the outer circumference of the rod (120).

## Patentansprüche

1. Arzneimittelinjektionsvorrichtung zur Verwendung bei der Hautchirurgie, umfassend:
eine Spritze (100) zum Injizieren von Arzneimittel in die Haut durch eine Nadel (130), die an einem Endabschnitt davon angeordnet ist;
eine Übertragungsführungsbaugruppe (200), die mit einem Zylinder (110) der Spritze (100) verbunden ist, in der Arzneimittel enthalten ist, wobei die Übertragungsführungsbaugruppe (200) ermöglicht, dass der Zylinder (110) eine Hubbewegung innerhalb eines begrenzten Bereiches gemäß einer Richtung, in der Arzneimittel injiziert wird, ausführt;
eine Injektionsbaugruppe (300), die an einer Seite der Übertragungsführungsbaugruppe (200) zur Bewegung einer Stange (120) des Zylinders (110) zu der Nadel (130) in Verriegelung mit der Übertragungsführungsbaugruppe (200) während einer Drehung durch eine Antriebskraft montiert ist; und
ein Steuerteil (400), das an einer Seite der Übertragungsführungsbaugruppe (200) und der Injektionsbaugruppe (300) montiert ist, wobei das Steuerteil (400) dazu dient, die Injektionsbaugruppe (300), wenn sich die Übertragungsführungsbaugruppe (200) zu einem Ende des begrenzten Bereiches bewegt, in einem Zustand zu betreiben, bei dem ein Endabschnitt der Nadel (130) in Kontakt mit der Haut kommt, und den Betrieb der Injektionsbaugruppe (300) zu stoppen, wenn der Endabschnitt der Nadel (130) von der Haut getrennt ist und sich die Übertragungsführungsbaugruppe (200) zu dem anderen Ende des begrenzten Bereiches bewegt,
wobei die Injektionsbaugruppe (300) die Stange (120) zu der Nadel (130) überträgt, solange der Zylinder (110) zu der Injektionsbaugruppe (300) zurückgezogen wird,
**dadurch gekennzeichnet, dass**
die Übertragungsführungsbaugruppe (200) umfasst:
eine Halterung (210), an der der Zylinder (110) fixiert ist;
einen Trägerblock (220), der an einem Stützsegment (222) montiert ist, das an einem vorderen Endabschnitt der Injektionsbaugruppe (300) montiert ist; und
eine Führungsschiene (230), die mit dem Boden der Halterung (210) verbunden und mit dem Trägerblock (220) gekoppelt ist, wobei die Führungsschiene (230) Sperreinrichtungen (232, 234) aufweist, die jeweils an vorderen und rückwärtigen Endabschnitten davon angeordnet sind, um die Halterung (210) hin- und herzubewegen,
wobei eine Distanz zwischen dem Trägerblock (220), der in Kontakt mit der Sperreinrichtung (232) auf einer Seite und der Sperreinrichtung (234) auf der anderen Seite kommt, gleich der Tiefe ist, die die Nadel (130) in die Haut eingeführt wird.

2. Arzneimittelinjektionsvorrichtung nach Anspruch 1, wobei die Injektionsbaugruppe (300) umfasst:
ein eine Antriebskraft übertragendes Element (310), das von dem Steuerteil (400) betrieben oder gestoppt wird und eine Antriebskraft an die Stange (120) überträgt;
ein Gehäuse (320), das einen Raum, in welchem das die Antriebskraft übertragende Element (310) eingebettet ist, sowie einen vorderen Endabschnitt aufweist, an dem die Übertragungsführungsbaugruppe (200) montiert ist; und
einen Bewegungswandler (330), der mit dem die Antriebskraft übertragenden Element (310) und der Stange (120) zur Umwandlung einer Drehbewegung in eine Hubbewegung gemäß einer Längsrichtung des Gehäuses (320) in Verriegelung mit dem die Antriebskraft übertragenden Element (310) und zur Bewegung der Stange (120) zu der Nadel verbunden ist.

3. Arzneimittelinjektionsvorrichtung nach Anspruch 1, wobei das Steuerteil (400) umfasst:
ein Erfassungsmittel (410), das an einem vorderen Endabschnitt der Übertragungsführungsbaugruppe (200) zur Bewegung der Übertragungsführungsbaugruppe (200) zu einem Ende des begrenzten Bereiches, wenn die Nadel (130) in Kontakt mit der Haut gelangt und in die Haut eingesetzt wird, oder zur Erfassung montiert ist, dass die Nadel (130) von der Haut getrennt wird; und
einen Controller (420), der an einer Seite der Injektionsbaugruppe (300) montiert und elektrisch mit dem Erfassungsmittel (410) und der Injektionsbaugruppe (300) verbunden ist, um so ein elektrisches Signal an die Injektionsbaugruppe (300) zu übertragen, so dass die Injektionsbaugruppe (300) gemäß dessen betrieben oder gestoppt wird, ob das Erfassungsmittel (410) das Signal erfasst oder nicht.

4. Arzneimittelinjektionsvorrichtung nach Anspruch 1, wobei die Übertragungsführungsbaugruppe (200) umfasst:
eine Skala (236), die an einem Rand der Führungsschiene (230) angeordnet ist; und
eine einstellbare Schraube (238), die mit der Führungsschiene (230) zum Fixieren der Sperreinrichtung (232, 234), die hin- und herbewegbar an der Führungsschiene (230) montiert ist, aus den beiden Sperreinrichtungen (232, 234) schraubgekoppelt ist, die an den vorderen und rückwärtigen Endabschnitten angeordnet sind.

5. Arzneimittelinjektionsvorrichtung nach Anspruch 2, wobei das die Antriebskraft übertragende Element (410) umfasst:
einen Antriebsmotor (312), der in das Gehäuse (320) eingebettet ist und vorwärts und rückwärts rotiert; und
eine Verlangsamungseinrichtung (314), die zwischen einer Antriebswelle des Antriebsmotors (312) und dem Bewegungswandler (330) montiert ist.

6. Arzneimittelinjektionsvorrichtung nach Anspruch 2, wobei das Gehäuse (320) umfasst:
einen Bewegungsschlitz (322), der an einer oberen Seite entlang einer Längsrichtung des Gehäuses (320) auf solche Weise durchlöchert ist, dass sich der Bewegungswandler (330) in dem Bewegungsschlitz (322) bewegt; und
ein Trägerfragment (324), das einteilig in das Gehäuse (320) zum Fixieren des die Antriebskraft übertragenden Elements (410) eingebettet ist.

7. Arzneimittelinjektionsvorrichtung nach Anspruch 2, wobei der Bewegungswandler (330) umfasst:
eine Übertragungsstange (332), die einen Endabschnitt, der mit einem Endabschnitt des die Antriebskraft übertragenden Elements (410) verbunden ist, um so vorwärts und rückwärts zu rotieren, wobei der andere Endabschnitt an dem Gehäuse (320) getragen ist, sowie eine mit Spiralgewinde versehene Außenumfangsfläche aufweist;
ein Bewegungsfragment (334), das mit der Übertragungsstange (332) schraubgekoppelt ist, so dass es sich entlang einer Längsrichtung der Übertragungsstange (332) hin- und herbewegt; und
ein Übertragungsfragment (336), das mit dem Bewegungsfragment (334) verbunden ist und zu dem oberen Abschnitt des Gehäuses (320) freiliegt, um so an der Stange anzuliegen.

8. Arzneimittelinjektionsvorrichtung nach Anspruch 7, wobei das Übertragungsfragment (336) umfasst:
einen Körper (337), der sich von einem Rand des Bewegungsfragments (334) erstreckt, wobei der Endabschnitt der Stange (120) an der Seite des Körpers (337) anliegt; und
ein Fixierfragment (339), das rotierend mit einem Endabschnitt des Körpers (337) verbunden ist, wobei das Fixierfragment (339) einen Fixierschlitz (339'), der an einem Endabschnitt davon geformt ist, auf solche Weise aufweist, dass eine Breite vorgesehen ist, die dem Außenumfang der Stange (120) entspricht.

## Revendications

1. Appareil d'injection de médicament à utiliser en chirurgie cutanée, comprenant :
une seringue (100) pour injecter un médicament dans la peau via une aiguille (130) disposée au niveau d'une portion terminale de celle-ci ;
un ensemble formant guide de transfert (200) réuni avec un cylindre (110) de la seringue (100) dans laquelle le médicament est contenu, l'ensemble formant guide de transfert (200) permettant au cylindre (110) d'effectuer un mouvement de va-et-vient dans une plage limitée selon une direction dans laquelle le médicament est injecté ;
un ensemble d'injection (300) monté sur un côté de l'ensemble formant guide de transfert (200) pour déplacer une tige (120) du cylindre (110) en direction de l'aiguille (130) en interverrouillage avec l'ensemble formant guide de transfert (200) tout en étant en rotation par une force d'entraînement ; et
une partie de commande (400) montée sur un côté de l'ensemble formant guide de transfert (200) et de l'ensemble d'injection (300), la partie de commande (400) servant à actionner l'ensemble d'injection (300) quand l'ensemble formant guide de transfert (200) se déplace vers une extrémité de la plage limitée dans un état dans lequel une portion terminale de l'aiguille (30) vient en contact avec la peau et à arrêter l'actionnement de l'ensemble d'injection (300) quand la portion terminale de l'aiguille (130) est séparée vis-à-vis de la peau, et l'ensemble formant guide de transfert (200) se déplace vers l'autre extrémité de la plage limitée,
dans lequel l'ensemble d'injection (300) transfère la tige (120) en direction de l'aiguille (130) aussi longtemps que le cylindre (110) se rétracte en direction de l'ensemble d'injection (300),
**caractérisé en ce que**
l'ensemble formant guide de transfert (200) comprend :
une monture (210) sur laquelle le cylindre (110) est fixé ;
un bloc de support (220) monté sur un segment en projection (222) monté au niveau d'une portion d'extrémité avant de l'ensemble d'injection (300) ; et
un rail guide (230) réuni au fond de la monture (210) et couplé avec le bloc de support (220), le rail guide (130) ayant des creux (232, 234) respectivement disposés au niveau de ses portions d'extrémité avant et arrière, de manière à déplacer la monture (210) en va-et-vient,
dans lequel une distance entre le bloc de support (220), qui vient en contact avec le creux (232) sur un côté, et le creux (234) sur l'autre côté est égale à la profondeur sur laquelle l'aiguille (130) est insérée dans la peau.

2. Appareil d'injection de médicament selon la revendication 1, dans lequel l'ensemble d'injection (300) comprend :
un élément de transfert de force d'entraînement (310) actionné ou arrêté par la partie de commande (400) et transférant une force d'entraînement à la tige (120) ;
un boîtier (320) ayant un espace dans lequel l'élément de transfert de force d'entraînement (310) est noyé et une portion d'extrémité avant sur laquelle l'ensemble formant guide de transfert (200) est monté ; et
un transducteur de mouvement (330) connecté à l'élément de transfert de force (310) et à la tige (120) pour convertir un mouvement rotatif en un mouvement de va-et-vient en accord avec une direction longitudinale du boîtier (320) en interverrouillage avec l'élément de transfert de force d'entraînement (310), et pour déplacer la tige (120) en direction de l'aiguille.

3. Appareil d'injection pour médicament selon la revendication 1, dans lequel la partie de commande (400) comprend :
des moyens de détection (410) montés au niveau d'une portion d'extrémité avant de l'ensemble formant guide de transfert (200) pour déplacer l'ensemble formant guide de transfert (200) vers une extrémité de la plage limitée quand l'aiguille (130) vient en contact avec la peau et est insérée dans la peau, ou pour détecter que l'aiguille (130) est séparée vis-à-vis de la peau ; et
un contrôleur (420) monté sur un côté de l'ensemble d'injection (300) et électriquement connecté avec les moyens de détection (410) et avec l'ensemble d'injection (300) de manière à transférer un signal électrique à l'ensemble d'injection (300), de sorte que l'ensemble d'injection (300) est actionné ou arrêté selon que les moyens de détection (410) détectent ou non le signal.

4. Appareil d'injection pour médicament selon la revendication 1, dans lequel l'ensemble formant guide de transfert (200) comprend :
une échelle (236) disposée sur un bord du rail guide (230) ; et
une vis ajustable (238) couplée par vissage avec le rail guide (230) pour fixer le creux (232, 234), qui est monté en va-et-vient sur le rail guide (230), hors des deux creux (232, 234) disposés au niveau des portions d'extrémité avant et arrière.

5. Appareil d'injection pour médicament selon la revendication 2, dans lequel l'élément de transfert de force d'entraînement (410) comprend :
un moteur d'entraînement (312) noyé dans le boîtier (320) et en rotation vers l'avant et vers l'arrière ; et
un élément de décélération (314) monté entre un arbre d'entraînement du moteur d'entraînement (312) et le transducteur de mouvement (330).

6. Appareil d'injection pour médicament selon la revendication 2, dans lequel le boîtier (320) comprend :
une fente de mouvement (322) perforée dans une face supérieure le long d'une direction longitudinale du boîtier (320), d'une manière telle que le transducteur de mouvement (330) se déplace dans la fente de mouvement (322) ; et
un fragment de support (324) intégralement noyé dans le boîtier (320) pour fixer l'élément de transfert de force d'entraînement (410).

7. Appareil d'injection pour médicament selon la revendication 2, dans lequel le transducteur de mouvement (330) comprend :
une barre de transfert (332) ayant une portion terminale réunie avec une portion terminale de l'élément de transfert de force d'entraînement (410) de manière à tourner vers l'avant et vers l'arrière, l'autre portion terminale étant supportée sur le boîtier (320), et une surface circonférentielle extérieure filetée en spirale ;
un fragment en mouvement (334) couplé par vissage avec la barre de transfert (332) de manière à se déplacer en va-et-vient le long d'une direction longitudinale de la barre de transfert (332) ; et
un fragment de transfert (336) réuni au fragment en mouvement (334) et exposé à la portion supérieure du boîtier (320) de manière à venir buter contre la tige.

8. Appareil d'injection pour médicament selon la revendication 7, dans lequel le fragment de transfert (336) comprend :
un corps (337) s'étendant depuis un bord du fragment en mouvement (334), la portion terminale de la tige (120) venant en butée sur le côté du corps (337) ; et
un fragment de fixation (339) réuni en rotation avec une portion terminale du corps (337), le fragment de fixation (39) ayant une fente de fixation (339') formée au niveau d'une portion terminale de celui-ci, de manière à avoir une largeur correspondant à la circonférence extérieure de la tige (120).
